# EUROPEAN PATENT APPLICATION

(11) **EP 1 695 973 A1**
(43) Date of publication of application: **30.08.2006**
(21) Application number: 05290428.1
(22) Date of filing: 24.02.2005
(51) Int. Cl.: C07D 471/04, A61K 31/519

(54) **Ocaperidone salt and pharmaceutical compositions containing the same**

(71) Applicant: NEURO3D, 68058 Mulhouse Cedex 2 (FR)
(72) Inventor: Biondi, Stefano, 68400 Riedisheim (FR); Demailly, Arnold, 68200 Mulhouse (FR); Mondadori, Cesare, 4153 Reinach (CH); Paparin, Jean-Laurent, 68720 Zillisheim (FR)
(74) Representative: Tezier Herman, Béatrice

(57) **Abstract**

The invention relates to a new salt of ocaperidone and its uses, particularly in the pharmaceutical industry. The invention discloses a specific salt of ocaperidone having increased water solubility, as well as therapeutic methods by administering said salt, in particular for treating various diseases of the central or peripheral nervous system, especially central nervous system. It further deals with pharmaceutical compositions comprising said salt and methods for preparing the same.

## Description

The invention relates to a new salt of ocaperidone and its uses, particularly in the pharmaceutical industry. The invention discloses a specific salt of ocaperidone having increased water solubility, as well as therapeutic methods by administering said salt, in particular for treating various diseases of the central or peripheral nervous system, especially central nervous system. It further deals with pharmaceutical compositions comprising said salt and methods for preparing the same.

European patent n° 0 453 042 A1 describes the 3-[2-[4-(6-fluoro-1,2-benzisoxazol-3-yl)-1-piperidinyl]ethyl]-2,9-dimethyl-pyrido-[1,2-a] pyrimidin-4-one also known as ocaperidone, a potent dopaminergic compound having particularly interesting antipsychotic properties. It has been described as an antagonist of neurotransmitters and in particular dopamine and serotonin. Therapeutic indications for using ocaperidone therefore are mainly in the CNS area, particularly as potent antipsychotic agents and more specifically for treating acute psychoses including schizophrenia, obsessive compulsive disorder (OCD), bipolar depression, and Tourette syndrome. Indeed, its combined dopamine-serotonin antagonism properties are especially interesting as they offer relief of both the positive and negative symptoms of schizophrenia.

Ocaperidone presents the following formula (I):

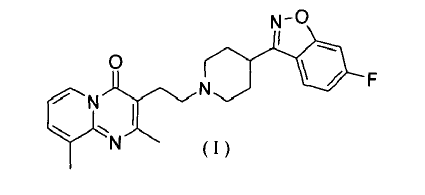

In view of its useful pharmacological properties, the subject compound is a candidate as a pharmacologically active ingredient. Water solubility is an essential parameter for the absorption of drugs and usually readily solubilized compounds are better absorbed by passive diffusion in the gastro-intestinal track. Unfortunately, ocaperidone as a free base form is poorly soluble in water (0.0007g/100ml) and also in acid solution (0.25 g/100m1 at pH = 1.3), which may result in variability in bioavailability. Acid addition salts of ocaperidone due to their increased water solubility may be advantageous over the corresponding base form in the preparation of pharmaceutical compositions.

The Applicant has now found that a particular salt of ocaperidone can be obtained in well defined reproducible amorphous and/or crystalline forms that especially exhibit valuable characteristics for formulation. The present invention deals with a specific salt of ocaperidone which shows very interesting water solubility. The release of such salt can be controlled in such a way that variability in bioavailability is reduced, absorption is optimized and plasma level are sustained over a long period of time. Moreover, said salt is perfectly reproducible, easily formulated and sufficiently stable to allow storage for long periods without particular requirements for temperature, light, humidity and/or oxygen level.

The present invention relates to an unexpected and exceptionally soluble (1:1) salt of ocaperidone in amorphous and/or crystalline forms, including all polymorphs forms of the salts. The salt of the present invention presents an organic acid moiety.

More specifically, the ocaperidone salt according to the invention presents an acid moiety which is pyroglutamic acid. The pyroglutamic acid is in the D- or L- form, or mixture thereof; it is more particularly the L-pyroglutamic acid form.

Ocaperidone salts presenting an acid moiety selected from succinic acid, fumaric acid, tartaric acid, N-(2-carboxyphenyl)-glycine acid, diglycolic acid, orotic acid, galactaric acid, nicotinic acid and hippuric acid were made for comparison. As shown in more details below, the ocaperidone salt solubility study shows that L-pyroglutamic acid addition salt is respectively 31.2 times more soluble than succinic acid addition salt which is 357 times more soluble than the ocaperidone free base form.

The compound according to the present invention may be prepared by various methods known to those skilled in the art. However, the ocaperidone salt of the invention is generally prepared by dissolution of ocaperidone and the organic acid as defined above, preferably in stoechiometric proportion, and advantageously in an organic solvent, for example, dichloromethane, methanol, ethanol, tetrahydrofuran, ethers or mixture thereof. The temperature of the process can vary upon a wide range, it is preferably comprised between 0°C and boiling temperature of the used solvents. When the salt is prepared, after a time comprised between a few seconds and several days, a crystalline product precipitates and can be isolated or collected by filtration, then can be washed with a cold (i.e. especially from 0°C to 25°C) organic solvent (preferably the same used for the crystallization step) and finally dried, preferably under vacuum.
In the crystallisation process according to the invention, it is possible to use the compound of formula (I), in particular as free base form, obtained by any process. Advantageously, the compound of formula (I) as free base form is obtained by the preparation process described in patent specification EP 0 453 042 is used.

In a particular embodiment of the process according to the invention, the concentration of ocaperidone in the solvent is preferably from 10-200 g/liter. The concentration of ocaperidone is preferably close to saturation.

It should be understood that other methods of producing this compound may be designed by the skilled person, based on common general knowledge and following guidance contained in this application.

According to a particular embodiment, the present invention relates to L-pyroglutamic acid addition salt of Formula (II). The salt of Formula (II) can be in amorphous and/or crystalline forms including all polymorphs of this compound.
Formula (II) is as follows :

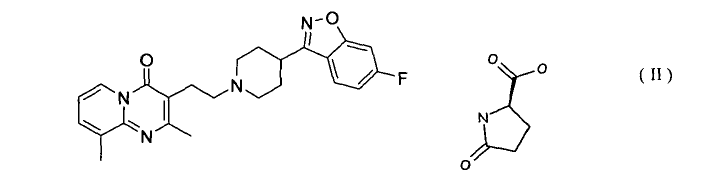

More specifically, the present invention relates to the crystalline form of the compound of formula (II), characterised by the following powder X-ray diffraction diagram (see below table (1)), measured using a X-ray diffractometer (DRX) Rigaku Miniflex (copper anticathode) and expressed in terms of inter-planar distance d (Å), Bragg's angle 2 theta (°), intensity (I) and relative intensity (expressed as a percentage of the most intense ray =Io):

**Table (1)**

| **Peak N°** | **2 theta (°)** | **d (Å)** | **Intensity I** | **I/Io** |
|---|---|---|---|---|
| **1** | **5.260** | **16.7863** | **845** | **8** |
| **2** | **11.040** | **8.0074** | **546** | **5** |
| **3** | **12.000** | **7.3689** | **990** | **9** |
| **4** | **13.380** | **6.6118** | **3917** | **34** |
| **5** | **15.000** | **5.9012** | **1196** | **11** |
| **6** | **16.140** | **5.4868** | **3023** | **26** |
| **7** | **17.960** | **4.9347** | **1637** | **14** |
| **8** | **19.040** | **4.6572** | **2551** | **22** |
| **9** | **20.160** | **4.4009** | **2768** | **24** |
| **10** | **21.440** | **4.1409** | **11687** | **100** |
| **11** | **22.360** | **3.9726** | **4965** | **43** |
| **12** | **23.280** | **3.8176** | **4877** | **42** |
| **13** | **23.980** | **3.7078** | **2645** | **23** |
| **14** | **25.000** | **3.5588** | **1761** | **16** |
| **15** | **26.600** | **3.3482** | **3955** | **34** |
| **16** | **28.160** | **3.1662** | **2479** | **22** |
| **17** | **28.480** | **3.1313** | **2197** | **19** |
| **18** | **29.980** | **2.9780** | **1951** | **17** |
| **19** | **31.640** | **2.8254** | **1843** | **16** |
| **20** | **32.620** | **2.7427** | **1685** | **15** |
| **21** | **33.560** | **2.6680** | **1044** | **9** |
| **22** | **34.180** | **2.6210** | **1034** | **9** |
| **23** | **35.420** | **2.5321** | **1312** | **12** |
| **24** | **36.160** | **2.4819** | **1260** | **11** |
| **25** | **36.960** | **2.4300** | **939** | **9** |
| **26** | **37.800** | **2.3779** | **1095** | **10** |
| **27** | **38.980** | **2.3086** | **1130** | **10** |
| **28** | **39.440** | **2.2828** | **1028** | **9** |
| **29** | **40.440** | **2.2286** | **1006** | **9** |
| **30** | **41.589** | **2.1701** | **1162** | **10** |
| **31** | **43.080** | **2.0979** | **1073** | **10** |
| **32** | **44.120** | **2.0509** | **1462** | **13** |
| **33** | **44.660** | **2.0273** | **1292** | **12** |
| **34** | **45.640** | **1.9860** | **1115** | **10** |
| **35** | **46.860** | **1.9371** | **1488** | **13** |
| **36** | **47.940** | **1.8960** | **1014** | **9** |
| **37** | **48.300** | **1.8827** | **1016** | **9** |
| **38** | **49.140** | **1.8524** | **1273** | **11** |
| **39** | **49.700** | **1.8329** | **1160** | **10** |
| **40** | **51.020** | **1.7885** | **1042** | **9** |
| **41** | **52.960** | **1.7275** | **1056** | **10** |

In a similar manner, nicotinic acid addition salt of ocaperidone (Formula (III)) was also prepared. The salt of Formula (III) can be in amorphous and/or crystalline forms including all polymorphs of this compound.
Formula (III) is as follows :

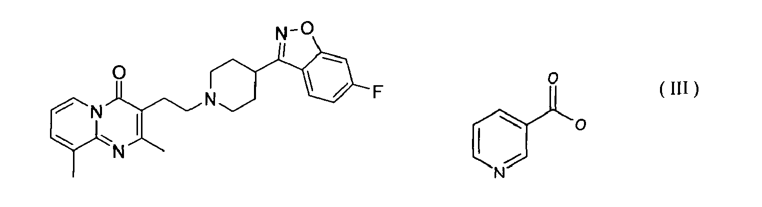

More specifically, the crystalline form of compound of formula (III) is characterised by the following powder X-ray diffraction diagram (see below table (2)), measured using a X-ray diffractometer (DRX) Rigaku Miniflex (copper anticathode) and expressed in terms of inter-planar distance d (Å), Bragg's angle 2 theta (°), intensity and relative intensity (expressed as a percentage of the most intense ray):

**Table (2)**

| **Peak N°** | **2 theta (°)** | **d (Å)** | **Intensity I** | **I/Io** |
|---|---|---|---|---|
| **1** | **11.060** | **7.9929** | **998** | **12** |
| **2** | **11.880** | **7.4430** | **927** | **11** |
| **3** | **12.520** | **7.0639** | **2480** | **29** |
| **4** | **13.300** | **6.6514** | **4841** | **57** |
| **5** | **14.720** | **6.0128** | **1226** | **15** |
| **6** | **16.420** | **5.3939** | **3993** | **47** |
| **7** | **17.360** | **5.1039** | **1209** | **15** |
| **8** | **18.900** | **4.6913** | **1911** | **23** |
| **9** | **20.120** | **4.4095** | **2031** | **24** |
| **10** | **21.240** | **4.1795** | **6837** | **80** |
| **11** | **22.180** | **4.0044** | **8591** | **100** |
| **12** | **23.100** | **3.8470** | **2534** | **30** |
| **13** | **23.940** | **3.7139** | **3596** | **42** |
| **14** | **25.020** | **3.5560** | **1512** | **18** |
| **15** | **25.660** | **3.4687** | **1318** | **16** |
| **16** | **26.420** | **3.3706** | **2100** | **25** |
| **17** | **27.600** | **3.2291** | **1737** | **21** |
| **18** | **28.480** | **3.1313** | **1285** | **15** |
| **19** | **29.600** | **3.0153** | **1288** | **15** |
| **20** | **30.040** | **2.9722** | **1236** | **15** |
| **21** | **31.360** | **2.8500** | **984** | **12** |
| **22** | **32.340** | **2.7658** | **959** | **12** |
| **23** | **33.980** | **2.6360** | **754** | **9** |
| **24** | **35.240** | **2.5446** | **1097** | **13** |
| **25** | **36.060** | **2.4886** | **736** | **9** |
| **26** | **37.860** | **2.3743** | **792** | **10** |
| **27** | **38.660** | **2.3270** | **744** | **9** |
| **28** | **39.460** | **2.2816** | **907** | **11** |
| **29** | **40.460** | **2.2275** | **941** | **11** |
| **30** | **41.560** | **2.1711** | **852** | **10** |
| **31** | **43.000** | **2.1016** | **1416** | **17** |
| **32** | **44.360** | **2.0403** | **967** | **12** |
| **33** | **45.500** | **1.9918** | **998** | **12** |
| **34** | **46.660** | **1.9450** | **1343** | **16** |
| **35** | **48.400** | **1.8790** | **859** | **10** |
| **36** | **49.340** | **1.8454** | **1029** | **12** |
| **37** | **54.940** | **1.6698** | **1401** | **17** |

In a similar manner, hippuric acid addition salt of ocaperidone (Formula (IV)) was also prepared. The salt of Formula (IV) can be in amorphous and/or crystalline forms including all polymorphs of this compound.
Formula (IV) is as follows :

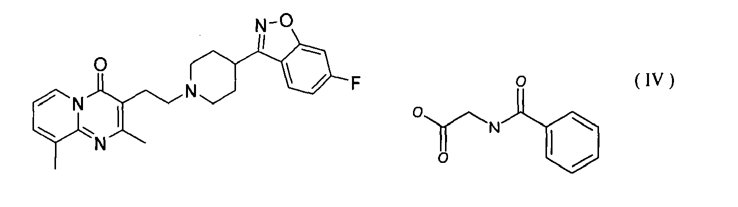

The present invention also relates to pharmaceutical compositions comprising at least one ocaperidone salt as defined above in a pharmaceutically acceptable vehicle or support, optionally in association with another active agent.

The pharmaceutical composition is more particularly intended to treat diseases of the central or peripheral nervous system, especially central nervous diseases, including psychosis. The present salt is particularly effective in treating acute psychoses including schizophrenia, obsessive compulsive disorder (OCD), bipolar depression, anxiety, mania, and Tourette syndrome. The present salt is more particularly effective in treating schizophrenia, including positive and negative symptoms of schizophrenia, e.g. anergy, apathy, social withdrawal and depressive mood, and also appear to reduce the incidence of extrapyramidal side-effects during maintenance therapy with classical neuroleptics, i.e. dopamine antagonists.

The present invention also relates to the use of an ocaperidone salt as defined above, for the preparation of a pharmaceutical composition for the treatment of diseases of the central or peripheral nervous system, in particular for the treatment of diseases as specified above.

The present invention also includes methods of treating diseases of the central or peripheral nervous system, in particular for the treatment of diseases as specified above, comprising the administration to a subject in need thereof of an effective amount of a ocaperidone salt as defined above.

As indicated above, a further object of this invention relates to a pharmaceutical composition comprising at least one ocaperidone salt as defined above, and a pharmaceutically acceptable vehicle or support.

The compound may be formulated in various forms, including solid and liquid forms, such as tablets, gel, syrup, powder, aerosol, etc. The ocaperidone salt can be formulated either in crystalline and/or amorphous forms.

The compositions of this invention may contain physiologically acceptable diluents, fillers, lubricants, excipients, solvents, binders, stabilizers, and the like. Diluents that may be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the compositions include but are not limited to starch, gelatin and fillers such as sucrose, glucose, dextrose and lactose.
Natural and synthetic gums that may be used in the compositions include but are not limited to sodium alginate, ghatti gum, carboxymethyl cellulose, methyl cellulose, polyvinyl pyrrolidone and veegum. Excipients that may be used in the compositions include but are not limited to microcrystalline cellulose, calcium sulfate, dicalcium phosphate, starch, magnesium stearate, lactose, and sucrose. Stabilizers that may be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.
Solvents that may be used include but are not limited to Ringers solution, water, distilled water, dimethyl sulfoxide to 50% in water, propylene glycol (neat or in water), phosphate buffered saline, balanced salt solution, glycol and other conventional fluids.

The dosages and dosage regimen in which the salts of the invention are administered will vary according to the dosage form, mode of administration, the condition being treated and particulars of the patient being treated. Accordingly, optimal therapeutic concentrations will be best determined at the time and place through experimentation.

The ocaperidone salt according to the invention can also be used enterally. Orally, the compounds according to the invention are suitably administered at the rate of 100 µg to 100 mg per day per kg of body weight. The required dose can be administered in one or more portions. For oral administration, suitable forms are, for example, tablets, gel, aerosols, pills, dragees, syrups, suspensions, emulsions, solutions, powders and granules; a preferred method of administration consists in using a suitable form containing from 0.01 mg to about 10 mg of active substance.

The compound according to the invention can also be administered parenterally in the form of solutions or suspensions for intravenous or intramuscular perfusions or injections. In that case, the compound according to the invention is generally administered at the rate of about 10 µg to 10 mg per day per kg of body weight; a preferred method of administration consists of using solutions or suspensions containing approximately from 0.01 mg to 1 mg of active substance per ml.

For the compound of this invention, the dose to be administered, whether a single dose, multiple dose, or a daily dose, will of course vary with the chosen route of administration, the size of the recipient, the type of disease and the nature of the patient's condition. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological and physiological effects. A doctor skilled in the art for treating the disease will be able to ascertain, without undue experimentation, appropriate protocols for the effective administration of the compound of this present invention, such as by referring to the earlier published studies on compounds found to have effect on the disease to be treated.

According to another aspect, the present invention relates to a method for the treatment of diseases of the central or peripheral nervous system, comprising administering to a patient in need of such treatment an effective amount of the ocaperidone salt as described above.

According to the invention, the term treatment denotes curative, symptomatic, and preventive treatment. Such ocaperidone salts, compositions comprising the same, or treatment can be implemented alone or in combination with other active ingredients, compositions or treatments. Moreover, it can correspond to treatment of chronic or acute disorders.

Formulations of the present invention suitable for oral administration may be presented as discrete units such as capsules, cachets or tablets each containing a predetermined amount of active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus or paste.

A tablet may be made by compression or moulding, optionally with one or more accessory ingredients. Compressed tablets may be prepared by compressing in a suitable machine the active ingredient in a free-flowing form such as powder or granules, optionally mixed with a binder, lubricant, inert diluents, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parental administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example saline or water-for-injection, immediately prior to use.

In a particular embodiment, composition of the invention is suitably formulated for a topical administration in the mouth, for example buccally or sublingually. In this particular embodiment, the ocaperidone salt of the invention can be in a crystalline and/or amorphous form, it is more particularly in an amorphous state. Said buccal or sublingual formulations include lozenges comprising the active ingredient in a flavored basis such as sucrose and acacia or tragacanth, pastilles comprising the active ingredient in a basis such as gelatin and glycerin or sucrose and acacia or any other form adapted to immediate release of the active ingredient in the mouth.

Extemporaneous injection solutions and suspensions may be prepared from sterile powders, granules and tablets of the kind previously described.

Formulations for rectal administration may be presented as a suppository with the usual carriers, such as cocoa butter or polyethylene glycol.

The relatively non-hygroscopic nature and the high solubility of the salt of ocaperidone of the invention renders them particularly suitable for administration in liquid and solid form. Preferred unit dosage formulations are those containing an effective dose, as herein before recited, or an appropriate fraction thereof, of the active ingredient. It should be understood that in addition to the ingredients particularly mentioned above, the formulations of this invention may include other agents conventional in the art having regard to the type of formulation in question, for example those suitable for oral administration may includes flavoring agents.

### FIGURES

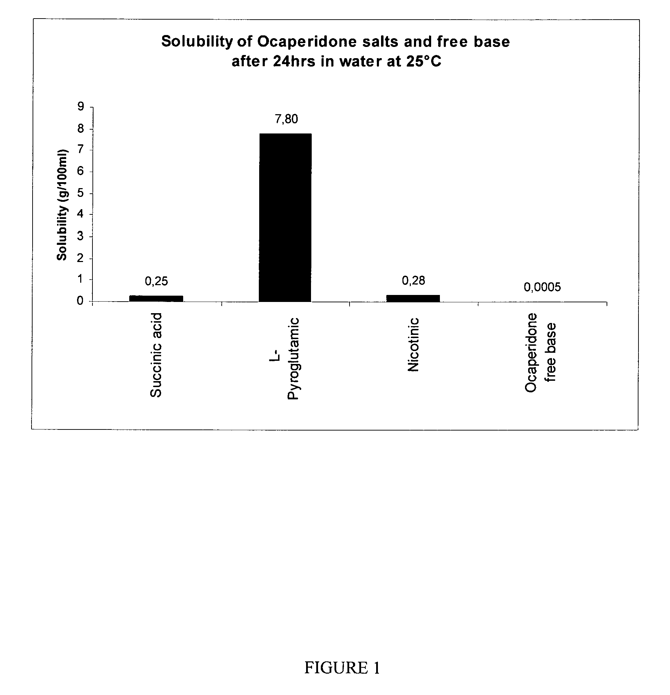

Figure 1 : Ocaperidone free base and salts solubility study in water at 25°C (24hours) in g/100ml

The invention is illustrated by the following examples. However, they are representative only and should not be construed as being limiting in any respect.

### EXAMPLES

The powder X-ray diffraction spectrum was measured under the following experimental conditions:
- DIFFRACTOMETER:: Rigaku MiniFlex
- DETECTOR SC-M:: Scintillator: NaI (T1)
Window material: Be
- X-RAY GENERATOR:: Copper anticathode (Lambda = 1,5405)
Tube output voltage: 30 kV
Tube output current: 15 mA
Kβ suppression filter: Ni-filter
- GONIOMETER:: Scanning axis: θ/2 θ interlocked
2 θ scanning range: -3° to +150°
Measurement range: +3° to +60°
Datum angle: 2θ =10°
Scattering: 4.2 deg.
Receiving: 0.3 mm
Scan speed: 2.00 s
Increment between each measurement: 0.02 deg.

### Experimental data processed using MiniFlex Program Manager Vers. 3.1

NMR spectra were obtained at 300 MHz on a Bruker AV 300 instrument using deuterated solvents. Chemical shifts are given in ppm relative to an internal standard of the solvent (ex: 4.79 for D2O).

### EXAMPLE 1

### Preparation of Ocaperidone succinic acid salt

100 mg (0.237 mmol) of ocaperidone and 28 mg of succinic acid were dissolved in 15 ml of an ethanol/THF (9/1) solution. The precipitated crystalline product is filtered, washed with cold ethanol and dried. The ocaperidone succinate salt (1/1) thus obtained melts at 184-185°C. ¹H NMR (300 MHz, CD₃OD + D₂O) : 8.83 (d, 1H), 7.90 (dd, 1H), 7.77 (d, 1H), 7.38 (dd, 1H), 7.30-7.15 (m, 2H), 3.82 (brd, 2H), 3.64-3.52 (m, 1H), 3.37-3.29 (m, 3H), 3.23-3.14 (m, 2H), 2.58 (s, 3H), 2.53 (s, 3H), 2.46 (s, 4H), 2.46-2.38 (m, 2H), 2.33-2.18 (m, 2H). Analysis calculated for the formula : C₂₈H₃₁FN₄O₆ C, 62.44 ; H, 5.54 ; N, 11.09. Found : C, 62.24 ; H, 5.80 ; N, 11.40.

### EXAMPLE 2

### Preparation of Ocaperidone pyroglutamic acid salt

1 g (2.37 mmol) of ocaperidone and 357 mg of L-pyroglutamic acid were dissolved in 10 ml of boiling THF. The precipitated crystalline product is filtered, washed with cold THF and dried. The ocaperidone pyroglutamate (1/1) thus obtained melts at 173-175°C. ¹H NMR (300 MHz, CD₃OD + D₂O) : 8.79 (d, 1H), 7.89 (dd, 1H), 7.74 (d, 1H), 7.35 (dd, 1H), 7.21 (t, 1H), 7.18 (dt, 1H), 4.06 (dd, 1H), 3.82 (brd, 2H), 3.56 (brt, 1H), 3.40-3.25 (m, 4H), 3.20-3.12 (m, 2H), 2.56 (s, 3H), 2.50 (s, 3H), 2.47-2.36 (m, 3H), 2.35-2.20 (m, 3H), 2.08-1.95 (m, 1H). Analysis calculated for the formula : C₂₉H₃₂FN₅O₅ : C, 63.37 ; H, 5.67 ; N, 12.74. Found : C, 62.98 ; H, 5.91 ; N, 12.84.

### EXAMPLE 3

### Preparation of Ocaperidone fumaric acid salt

Following example 1, using fumaric acid instead of succinic acid, ocaperidone fumaric acid salt is isolated. m.p. = 204-206° C. ¹H NMR (300 MHz, CD₃OD + D₂O) : 8.81 (d, 1H), 7.86 (dd, 1H), 7.74 (d, 1H), 7.36 (d, 1H), 7.25-7.10 (m, 2H), 6.56 (s, 2H), 3.80 (brs, 2H), 3.54 (brs, 1H), 3.35-3.25 (m, 4H), 3.17-3.07 (m, 2H), 2.54 (s, 3H), 2.50 (s, 3H), 2.46-2.34 (m, 2H), 2.29-2.12 (m, 2H). Analysis calculated for the formula : C₂₈H₂₉FN₄O6 : C, 62.68 ; H, 5.45 ; N, 10.44. Found : C, 62.21 ; H, 5.77 ; N, 11.25.

### EXAMPLE 4

### Preparation of Ocaperidone tartaric acid salt

Following example 1, using tartaric acid instead of succinic acid, ocaperidone tartaric acid salt is isolated. m.p. = 200-202° C. ¹H NMR (300 MHz, CD₃OD + D₂O) : 8.79 (d, 1H), 7.83 (m, 1H), 7.75 (d, 1H), 7.36 (d, 1H), 7.25-7.10 (m, 2H), 4.32 (s, 4H), 3.85 (brs, 2H), 3.52 (brs, 2H), 3.35-3.25 (m, 4H), 3.17-3.07 (m, 2H), 2.53 (s, 3H), 2.50 (s, 3H), 2.46-2.34 (m, 2H), 2.29-2.12 (m, 2H).

### EXAMPLE 5

### Preparation of Ocaperidone N-(2-carboxyphenyl)-glycine acid salt

Following example 2, using N-(2-carboxyphenyl)-glycine acid instead of L-pyroglutamic acid, ocaperidone N-(2-carboxyphenyl)-glycine acid salt is isolated. m.p. = 184-186° C. ¹H NMR (300 MHz, CD₃OD + D₂O) : 8.80 (d, 1H), 7.86 (dd, 1H), 7.73 (m, 2H), 7.34 (dd, 1H), 7.26-7.10 (m, 3H), 6.54-6.43 (m, 2H), 3.74 (brs, 2H), 3.66 (s, 2H), 3.50 (brs, 1H), 3.17-3.07 (m, 2H), 2.53 (s, 3H), 2.48 (s, 3H), 2.43-2.32 (m, 2H), 2.25-2.13 (m, 2H). Analysis calculated for the formula : C₃₃H₃₄FN₅O₆ : C, 64.38; H, 5.57 ; N, 11.38. Found : C, 64.10 ; H, 5.38 ; N, 12.13.

### EXAMPLE 6

### Preparation of diglycolic acid salt

Following example 2, using diglycolic acid instead of L-pyroglutamic acid, ocaperidone diglycolic acid salt is isolated. m.p. = 158-160° C. ¹H NMR (300 MHz, CD₃OD + D₂O) : 8.91 (d, 1H), 7.97 (dd, 1H), 7.85 (dd, 1H), 7.35-7.23 (m, 2H), 4.09 (s, 4H), 3.96 (brs, 2H), 3.63 (brs, 1H), 3.43-3.36 (m, 4H), 3.29-3.22 (m, 2H), 2.64 (s, 3H), 2.61 (s, 3H), 2.57-2.46 (m, 2H), 2.37-2.22 (m, 2H). Analysis calculated for the formula : C₂₈H₃₁FN₄O₇ : C, 60.64 ; H, 5.63 ; N, 10.10. Found : C, 61.26 ; H, 5.22 ; N, 10.46.

### EXAMPLE 7

### Preparation of Ocaperidone galactaric acid salt

Following example 2, using galactaric acid instead of L-pyroglutamic acid, ocaperidone galactaric acid salt is isolated. m.p. = 180-182° C. ¹H NMR (300 MHz, CD₃OD + D₂O) : 8.81 (d, 1H), 7.87 (dd, 1H), 7.76 (d, 1H), 7.38 (dd, 1H), 7.23 (t, 1H), 7.18 (dt, 1H), 4.15 (s, 1H), 3.86 (s, 1H), 3.84 (brs, 2H), 3.55 (brs, 1H), 3.33-3.26 (m, 2H), 3.18-3.10 (m, 2H), 2.56 (s, 3H), 2.51 (s, 3H), 2.46-2.35 (m, 2H), 2.27-2.09 (m, 2H).

### EXAMPLE 8

### Preparation of Ocaperidone orotic acid salt

Following example 2, using orotic acid instead of L-pyroglutamic acid, ocaperidone orotic acid salt is isolated. m.p. = 245-246° C. ¹H NMR (300 MHz, CD₃OD + D₂O) : 8.80 (d, 1H), 7.87 (dd, 1H), 7.77 (d, 1H), 7.38 (dd, 1H), 7.25 (t, 1H), 7.18 (brt, 1H), 6.04 (s, 1H), 3.75 (brs, 2H), 3.53 (brs, 1H), 3.18-3.10 (m, 2H), 2.55 (s, 3H), 2.51 (s, 3H), 2.45-2.35 (m, 2H), 2.26-2.10 (m, 2H).

### EXAMPLE 9

### Preparation of Ocaperidone hippuric acid salt

Following example 2, using hippuric acid instead of L-pyroglutamic acid, ocaperidone hippuric acid salt is isolated. m.p. = 163-165° C. ¹H NMR (300 MHz, CD₃OD + D₂O): 8.77 (d, 1H), 7.82 (dd, 1H), 7.76 (m, 2H), 7.62 (d, 1H), 7.41 (m, 1H), 7.37-7.29 (m, 3H), 7.13-7.06 (m, 2H), 3.87 (s, 1H), 3.59 (brd, 2H), 3.37 (m, 1H), 3.04 (s, 4H), 2.94 (t, 2H), 2.50 (s, 3H), 2.47 (s, 3H), 2.26 (dd, 2H), 2.20-2.06 (m, 2H).

### EXAMPLE 10

### Preparation of Ocaperidone nicotinic acid salt

Following example 2, using nicotinic acid instead of L-pyroglutamic acid, ocaperidone nicotinic acid salt is isolated. m.p. = 160-161° C. ¹H NMR (300 MHz, CD₃OD + D₂O) : 8.93 (d, 1H), 8.82 (d, 1H), 8.53 (dd, 1H), 8.22 (brd, 1H), 7.92 (dd, 1H), 7.75 (d, 1H), 7.44 (t, 1H), 7.40 (dt, 1H), 7.23 (t, 1H), 7.17 (dt, 1H), 3.89 (brs, 2H), 3.61 (brs, 1H), 3.45-3.33 (m,3H), 3.26-3.17 (m, 2H), 2.62 (s, 3H), 2.49 (s, 3H), 2.47-2.40 (m, 2H), 2.38-2.23 (m, 2H).

### EXAMPLE 11

### General procedure for the preparation of amorphous salt

A 1:1 mixture of ocaperidone (0.5 mmol) and the desired acid (0.5 mmol) is added into 8ml of water and 2ml of THF to get an homogeneous solution. The obtained mixture is freezed in a cold bath (at -78°C) and put to lyophilize overnight to obtain a fluffy white solid.

### EXAMPLE 12

### Solubility of ocaperidone salts in water

Ocaperidone pyroglutamic acid salt of example 2 presents a higher solubility in water than the other salts described in the examples 1, 3-10 and ocaperidone free base.
In particular, the solubility of ocaperidone salts was measured under the following experimental conditions:
UV/visible spectrophotometer : Agilent 8453E
Balance : Mettler Toledo AX205
Volumetric flask (5ml, 10 ml, 20ml, 100ml)
Mechanical pipettes : Biohit (m1000, m200)
Thermostat : Lauda E112T
Magnetic stirrer : Heidolph MR3001K

A solution of 100mg of crystalline ocaperidone salt or ocaperidone free base in 1 ml HPLC grade pure water (pH = 6) is prepared. The solution is kept at fixed and controlled temperature (ex : 25°C) under magnetic stirring for 24 hours. A sample is taken, filtered and the solubility of the ocaperidone salts or ocaperidone free base is measured after 1hour and 24h. Results are given in g/100ml.

The obtained results are presented in figure 1. These results show the unexpectedly high solubility of ocaperidone pyroglutamic salt.

## Claims

1. A salt of ocaperidone in amorphous and/or crystalline forms, including all polymorphs of this compound, said salt presenting an acid moiety which is pyroglutamic acid.

2. A salt according to claim 1, wherein the pyroglutamic acid is in the D-, L- form or mixture thereof.

3. A L-pyroglutamic acid addition salt of ocaperidone of the following formula (II) :

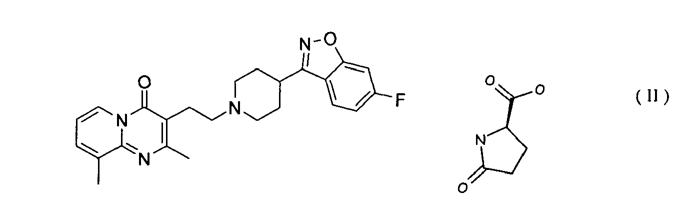

4. A pharmaceutical composition comprising at least one salt as defined in one of the preceding claims, and a pharmaceutically acceptable vehicle or support.

5. A pharmaceutical composition according to claim 4, intended to treat diseases of the central or peripheral nervous system, especially central nervous diseases, including psychosis.

6. A pharmaceutical composition according to claim 5, intended to treat schizophrenia, obsessive compulsive disorder (OCD), bipolar depression, anxiety, mania, or Tourette syndrome.

7. A pharmaceutical composition according to claim 5 or 6, which is suitable for a buccal or sublingual formulation.

8. A method for preparing a salt as defined in any of the preceding claims 1-3, including dissolution of ocaperidone and pyroglutamic acid, preferably in stoechiometric proportion, and advantageously in an organic solvent, and isolation of the obtained salt.
